# FASCICULE DE BREVET EUROPEEN

(11) **EP 0 373 046 B1**
(45) Date de publication et mention de la délivrance du brevet: **14.07.1993**
(21) Numéro de dépôt: 89403325.7
(22) Date de dépôt: 30.11.1989
(51) Int. Cl.: A61K 7/48, A61K 9/107, A61K 47/00

(54) **Crème dermatologique auto-émulsionnable**
Selbstemulgierbare dermatologische Creme
Self-emulsifiable dermatological cream

(30) Priorité: 02.12.1988 FR 8815827; 15.06.1989 FR 8907953
(43) Date de publication de la demande: 13.06.1990
(73) Titulaire: Grimberg, Georges Serge, F-75007 Paris (FR)
(72) Inventeur: Grimberg, Georges Serge, F-75007 Paris (FR)
(74) Mandataire: Madeuf, Claude Alexandre Jean

(56) Documents cités:
- BE-A- 869 644
- FR-A- 2 258 165
- GB-A- 2 069 333
- US-A- 2 129 836
- US-A- 4 375 480
- CHEMICAL ABSTRACTS, vol. 86, no. 6, 7 février 1977, pages 321-322, résumé no. 34591j, Columbus, Ohio, US; M.J. GROVES et al.: "Rheological characterisation of self-emulsifying oil/surfactant systems", & ACTA PHARM. SUEC. 1976, 13(4), 353-60

## Description

Toutes les crèmes utilisées en cosmétologie ainsi que la majorité des pommades pharmaceutiques contiennent deux phases l'une aqueuse, l'autre huileuse. C'est le cas, en particulier, de la crème hypoallergénique faciale décrite dans le US - A - 4 375 480 dont le mode de préparation par mélange des deux phases se fait entre 70 et 40°C avec agitation modérée, cette crème destinée à la peau peut contenir également de la vitamine E.

La présente invention a pour objet une crème dermatologique auto-émulsionnable dont les composants sont mélangés à température ambiante puis le tout est chauffé à une température convenable pour la fusion et la solubilisation de tous les produits, puis on agite en laissant refroidir et on ajoute ensuite à une température relativement basse tout parfum et additif approprié sensible à la chaleur.

On note également que la crème de base ci-dessus peut être complétée par les parfums et/ou les produits actifs divers.

Ces différents produits sont des parfums et/ou des principes actifs tels que des levures simples ou composées comme une levure magnésienne, des antibiotiques, des lyophilisats biologiques ainsi que tous produits compatibles avec la crème et susceptibles d'assouplir la peau, de la soigner ou de pénétrer parfaitement dans l'organisme.

Conformément à l'invention, après avoir mélangé les produits à température ambiante qui sont :
- Monostéarate de sorbitane
- Monostéarate de sorbitane polyoxy éthyléné,
- Acide stéarique
- Huile de vaseline
- Myristate d'isopropyle
- Triéthanolamine
- Para hydroxy benzoate de méthyle
- Para hydroxy benzoate de propyle
- Eau (distillée ou naturelle)

on chauffe et, ce, jusqu'à fusion et solubilisation de tous les produits sus-énoncés et on porte à une température convenable comprise entre 80°C et 120°C, plus spécialement entre 100°C et 105°C, pendant 15 à 75 min puis on agite le tout en laissant refroidir.

Suivant une autre caractéristique de l'invention, les parfums et additifs appropriés sensibles à la chaleur sont ajoutés à une température relativement basse comprise entre 25°C et 50°C, plus spécialement entre 35°C et 40°C.

Diverses autres caractéristiques de l'invention ressortent d'ailleurs de la description détaillée qui suit.

A titre d'exemple, on peut citer la formule suivante :
- Monostéarate de sorbitane 8,55 g
- Monostéarate de sorbitane polyoxyéthyléné 8,55 g
- Acide stéarique 31,8 g
- Huile de vaseline 17,1 g
- Myristate d'isopropyle 42,0 g
- Levure magnésienne 4,0 g
- Triéthanolamine O,424 g
- Para hydroxy benzoate de méthyle 0,159 g
- Para hydroxy benzoate de propyle O,159 g
- Eau distillée 283,5 g
- Parfum 1,720 g

Toutes les matières ci-dessus, sauf le parfum, sont mises à chauffer jusqu'à fusion complète des produits ; on soumet ensuite le mélange à une agitation et on ajoute le parfum vers 40°C.

Par chauffage du mélange jusqu'à fusion et solubilisation de tous les produits sus-énoncés, on entend porter ce mélange à une température convenable comprise entre 80°C et 120°C, plus spécialement entre 100°C et 105°C, pendant 15 à 75 min puis on agite le tout en laissant refroidir.

Les parfums et additifs appropriés sensibles à la chaleur sont ajoutés à une température relativement basse comprise entre 25°C et 50°C, plus spécialement entre 35°C et 40°C.

Le temps de chauffage peut être évalué, en général, à 1/2 heure.

Une telle crème utilisée en application tous les matins a été testée sur un échantillon de dix femmes de 25 à 80 ans et sur trois hommes de 50 à 55 ans.

Les résultats obtenus sont surtout remarquables à deux points de vue :
1) la crème est parfaitement tolérée et on n'a observé aucune réaction d'échauffement ou d'irritation même oculaire,
2) la crème assouplit la peau et diminue très nettement les rides. Les trois hommes de l'échantillon ont également appliqué cette crème avant de faire des trajets en motocyclettes de 150 à 450 km. Là encore les résultats ont été excellents, la peau est restée souple et non déshydratée.

Indépendamment de sa qualité auto-émulsionnable, la crème dermatologique conforme à l'invention peut se diluer pour donner un lait, par simple addition d'eau quand la crème est refroidie et maintenue au stockage. Cette dilution peut également avoir lieu à chaud.

En outre, et indépendamment de sa qualité auto-émulsionnable et de cette possibilité de dilution, la crème dermatologique conforme à l'invention est, en fonction des quantités de produits mis en oeuvre, thixotrope, c'est-à-dire qu'en l'agitant, elle redevient liquide puis reprend son aspect crémeux un certain temps après l'arrêt de l'agitation. Il est donc facile d'incorporer à cette crème et sous agitation à nouveau d'autres produits extemporanément, par exemple des antibiotiques, des antimycosiques et, après arrêt de l'agitation, elle redevient crémeuse.

Le produit encore liquide est, par exemple, mis dans des ampoules de 10 ml. Le produit devient ensuite solide et, quand on agite l'ampoule, le produit redevient liquide et il est possible de le récupérer et d'y ajouter tous produits appropriés.

A titre d'exemple, 10 ml d'une crème parfumée ou non qui se trouvait dans une ampoule à deux pointes ont été agités très rapidement. La crème est alors devenue liquide. Cette crème liquide, versée dans un pulvérisateur compte-gouttes qui contenait de la polymyxine, de l'oxytétracycline, de la nystatine et de la dexaméthasone, a donné après agitation un produit fluide qui, au bout d'un certain temps, est redevenu solide.

L'utilisation d'une telle crème-solide liquide, à usage non plus cosmétologique mais pharmaceutique, a été étudiée pour une mise en place dans les cavités sinusiennes et/ou auriculaires. Sous forme liquide, la crème s'étale au niveau de la muqueuse et, avec le temps, elle se fixe à la muqueuse en se solidifiant. Les résultats cliniques obtenus ont été excellents et ont permis de soigner de nombreuses infections de la sphère ORL.

## Revendications

1. Crème dermatologique auto-émulsionnable, caractérisée en ce que après avoir mélangé les produits à température ambiante qui sont :
- Monostéarate de sorbitane
- Monostéarate de sorbitane polyoxy éthyléné,
- Acide stéarique
- Huile de vaseline
- Myristate d'isopropyle
- Triéthanolamine
- Para hydroxy benzoate de méthyle
- Para hydroxy benzoate de propyle
- Eau (distillée ou naturelle)
on chauffe et, ce, jusqu'à fusion et solubilisation de tous les produits sus-énoncés et on porte à une température convenable comprise entre 80°C et 120°C, plus spécialement entre 100°C et 105°C, pendant 15 à 75 min puis on agite le tout en laissant refroidir.

2. Crème suivant la revendication 1, caractérisée en ce que les parfums et additifs appropriés sensibles à la chaleur sont ajoutés à une température relativement basse comprise entre 25°C et 50°C, plus spécialement entre 35°C et 40°C.

3. Crème selon les revendications 1 et 2, caractérisée en ce que de l'eau est ajouté à froid ou à chaud, afin de la diluer.

4. Crème selon les revendications 1 à 3, caractérisée en ce que différents produits peuvent y être ajoutés à la température qui convient, tels que :
- des parfums
- des levures simples ou composées et par exemple une levure magnésienne
- des antibiotiques solubles en phase aqueuse ou huileuse ou insolubles
- des antimycosiques
- des lyophilisats biologiques
- tous produits compatibles avec la crème et susceptibles d'assouplir la peau, de la soigner ou de pénétrer profondément dans l'organisme.

5. Crème selon les revendications 1 à 4, caractérisée par la formule de base suivante :
- Monostéarate de sorbitane 8,55 g
- Monostéarate de sorbitane polyoxyéthyléné 8,55 g
- Acide stéarique 31,8 g
- Huile de vaseline 17,1 g
- Myristate d'isopropyle 42,0 g
- Levure magnésienne 4,0 g
- Triéthanolamine O,424 g
- Para hydroxy benzoate de méthyle 0,159 g
- Para hydroxy benzoate de propyle O,159 g
- Eau distillée 283,5 g
- Parfum 1,720 g

## Patentansprüche

1. Selbstemulgierbare dermatologische Creme, dadurch gekennzeichnet, daß man nach der Mischung bei Raumtemperatur der folgenden Produkte:
- Sorbitanmonosterat
- Sorbitan-Polyoxy-Ethylen-Monosterat
- Stearinsäure
- Vaselineöl
- Isopropyl-Myristat
- Thriethanolamin
- Methyl-Parahydroxy-Benzonat
- Propyl-Parahydroxy-Benzonat
- Wasser (destilliertes oder natürliches)
das Ganze weiter bis zum Schmelzen und Auflösen aller oben genannten Produkte erhitzt, für 75 Minuten eine geeignete Temperatur zwischen 80° C und 120° C, vorzugsweise zwischen 100° c und 105° C geschaffen wird, und sodann das Ganze während des Abkühlens geschüttelt wird.

2. Creme nach Anspruch 1, dadurch gekennzeichnet, daß die geeigneten Parfums und Zusatzstoffe, die hitzeempfindlich sind, bei relativ niedriger Temperatur zwischen 25° C und 50° C, vorzugsweise zwischen 35° C und 40° C hinzugefügt werden.

3. Creme nach den Ansprüchen 1 und 2, dadurch gekennzeichnet, daß im kalten oder erhitzen Zustand Wasser zu ihrer Verdünnung beigegeben wird.

4. Creme nach den Ansprüchen 1 bis 3, dadurch gekennzeichnet, daß ihr verschiedene Produkte bei einer jeweils geeigneten Temperatur hinzugegeben werden können wie:
- Parfums
- einfache oder zusammengesetzte Hefen, zum Beispiel eine magnesiumhaltige Hefe
- lösliche Antibiotika in der wässrigen oder öligen Phase oder Nicht-lösliche Antibiotika
- Antimycotika
- biologische Lyophilisate
- jegliche mit der Creme kompatiblen Produkte, die die haut geschmeidig machen und sie behandeln können, oder tief in den Organismus eindringen können.

5. Creme nach den Ansprüchen 1 bis 4, gekennzeichnet durch die folgende Grundformel:
- Sorbitanmonosterat 8,55 g
- Sorbitan-Polyoxy-Ethylen-Monosterat 8,55 g
- Stearinsäure 31,8 g
- Vaselineöl 17,1 g
- Isopropylmyristat 42,0 g
- Magnesiumhaltige Hefe 4,0 g
- Thriethanolamin 0,424g
- Methyl-Parahydroxy-Benzonat 0,159g
- Propyl-Parahydroxy-Benzonat 0,159g
- destilliertes Wasser 283,5 g
- Parfum 1,720g

## Claims

1. Self-emulsifiable dermatologic cream, characterized in that, after having mixed the products at room temperature, which are
- Sorbitan monostearate
- Polyoxyethylenated sorbitan monostearate
- Stearic acid
- Petrolatum oil
- Isopropyl myristate
- Triethanolamine
- Methyl parahydroxybenzoate
- Propyl parahydroxybenzoate
- water (distilled or natural)
there is proceeded to heating until melt and solubilisation of all the above mentioned products, and bringing to a convenient temperature comprised between 80°C and 120°C, more especially between 100°C and 105°C, for 15 to 75 minutes, then the whole is stirred while letting it to cool down.

2. Cream according to claim 1, characterized in that the appropriate heat sensitive fragrances and additives are added at a relatively low temperature between 25°C and 50°C, more especially between 35°C and 40°C.

3. Cream according to claims 1 and 2, characterized in that water is added in the cold or hot state in order to dilute it.

4. Cream according to claims 1 to 3, characterized in that various products can be added thereto at a convenient temperature, such as :
- fragrances ,
- simple or compounded yeasts and for example a magnesian yeast,
- antibiotics which are soluble in aqueous or oily phase, or insoluble,
- antimycosics,
- biological lyophilizates,
- all products compatible with the cream and likely to soften the skin, to treat it or to penetrate deeply the system.

5. Cream according to claims 1 to 4, characterized by the following basic formula :
- Sorbitan monostearate 8.55 g
- Polyoxyethylenated sorbitan monostearate 8.55 g
- Stearic acid 31.8 g
- Petrolatum oil 17.1 g
- Isopropyl myristate 42.0 g
- Magnesian yeast 4.0 g
- Triethanolamine 0.424 g
- Methyl parahydroxybenzoate 0.159 g
- Propyl parahydroxybenzoate 0.159 g
- Distilled water 283.5 g
- Fragrance 1.720 g
